# EUROPEAN PATENT SPECIFICATION

(11) **EP 1 923 079 B1**
(45) Date of publication and mention of the grant of the patent: **20.07.2011**
(21) Application number: 07254446.3
(22) Date of filing: 14.11.2007
(51) Int. Cl.: A61L 27/30

(54) **Articular prothesis with a metallic part coated with wear resistant ceramic**
Gelenkprothese mit einem metallischen Teil, welches mit einer verschleissfesten Keramik beschichtet ist
Prothèse articulaire comprenant une partie métallique recouverte d'une céramique résistante à l'usure

(30) Priority: 16.11.2006 GB 0622896
(43) Date of publication of application: 21.05.2008
(73) Proprietor: Biomet UK Limited, Bridgend South Wales CF31 3XA (GB)
(72) Inventor: Khan, Mohammed Imran, Dr., Reading Berkshire RG1 3PP (GB); Scott, Robert Andrew, Chippenham Wiltshire SN15 4BX (GB); BIGSBY, Robert John Andrew, Penarth South Wales CF64 1EJ (GB)
(74) Representative: Giles, Ashley Simon

(56) References cited:
- WO-A1-2005/070344
- US-A- 5 123 924
- US-A1- 2005 164 041
- US-A1- 2006 184 251
- NARAYAN R J: "Nanostructured diamondlike carbon thin films for medical applications" MATERIALS SCIENCE AND ENGINEERING C, ELSEVIER SCIENCE S.A, CH, vol. 25, no. 3, May 2005 (2005-05), pages 405-416, XP004967309 ISSN: 0928-4931
- MÜNZ W-D: "Large-Scale Manufacturing of Nanoscale Multilayered Hard Coatings Deposited by Cathodic Arc/Unbalanced Magnetron Sputtering" MRS BULLETIN, PITTSBURGH, US, vol. 28, no. 3, March 2003 (2003-03), pages 173-179, XP002301638

## Description

This invention relates to a prosthesis and particularly but not exclusively relates to a prosthesis having a metallic component, at least a portion of a surface of which comprises a ceramic material.

### BACKGROUND

Joint replacement surgery involves the removal and replacement of diseased or damaged tissue from the articulating bones of a joint and the implantation of an artificial prosthesis to replace the removed tissue.

A total joint replacement (TJR) prosthesis comprises two components having bearing surfaces that are formed from wear resistant materials. The most common bearing arrangement comprises a metal component articulating against a plastic bearing component. Generally, the metal component is manufactured from cobalt chromium molybdenum alloy and the plastic component is made from ultra high molecular weight polyethylene (UHMWPE). It has been recognised that the production of UHMWPE debris at the articulating surfaces of joint replacements can play a major role in loosening and failure of these joints. As a result, metal on metal (MOM) and ceramic on ceramic (COC) articulations have been developed. These bearing combinations have the potential to greatly reduce wear of replacement joints, thus increasing the lifetime of such joints. However, each of these bearing combinations carries associated disadvantages.

MOM bearings display excellent mechanical properties and low wear rates. However, there are concerns over the risks associated with long term metal ion exposure. MOM bearings produce smaller sized and larger numbers of wear particles than polyethylene. Due to their size, these particles may be more readily dissolved than polyethylene particles, resulting in increased metal ion levels.

COC bearings posses extremely low wear rates and both the bearings and their wear particles are stable to biodegradation. However, the inherent brittle nature of ceramics leads to a risk of in vivo brittle fracture of the prosthesis.

A metal on ceramic (MOC) articulation is disclosed in WO01/017464. The MOC articulation addresses many of the disadvantages associated with COC articulations, and the difference in hardness between the components reduces wear and wear particle generation. However, this articulation still carries the risks associated with long term metal ion release.

US 2006/0184251 A1 discloses a medical device comprising a structural member, wherein at least a portion of a surface of the structural member is coated with a nanostructured material, such as a ceramic, to a thickness of at least about 25 micrometers.

W02005/070344 discloses a medical device, the surface of which is treated by exposure to a gas at a pressure less than atmospheric pressure to produce a hardened, minimally abrasive surface layer substantially comprising chromium nitride.

### SUMMARY OF INVENTION

According to the present invention, there is provided a bearing, such as a prosthetic joint comprising a first component and a second component, the first component being adapted to articulate with the second component and being formed from a ceramic material, and the second component being formed from a metallic material, wherein at least a portion of a surface of the second component comprises a coating of ceramic material of 2 to 12 microns in thickness applied to the second component, the coating comprising a multilayer superlattice coating applied by high power impulse magnetron sputtering.

It is an advantage of the invention that the second component has the increased toughness associated with metallic materials, but the bearing demonstrates the very low wear rates associated with a ceramic on ceramic articulation. Further, the second component, being formed primarily from metal, may be formed in a greater range of sizes than a similar component formed from ceramic.

It is a further advantage of the invention that the metallic component does not present a metallic surface, so as to reduce tissue exposure to metal ions.

The first and second components may comprise bearing surfaces. At least a portion of the bearing surface of the second component may comprise a coating of ceramic material.

The entire bearing surface of the second component may comprise a coating of ceramic material.

The entire surface of the second component may comprise a ceramic material. The first component may be formed from a material selected from the group comprising: alumina, stabilised zirconia, zirconia toughened alumina, silicon nitride and silicon carbide.

The second component may be formed from a material selected from the group comprising: cobalt chromium based alloys, titanium alloys and stainless steels.

The portion of a surface of the second component may comprise a coating of a chromium compound.

The coating may be formed from one or more materials selected from the group comprising: diamond like carbon, titanium nitride, titanium niobium nitride, titanium carbonitride, chromium nitride, chromium carbonitride, chromium carbide, chromium oxide, titanium aluminium nitride, niobium nitride and niobium carbide.

The prosthesis may comprise a hip replacement prosthesis. If the first component comprises a femoral head and the second component comprises an acetabular cup, then, for a given size of acetabulum in total hip replacement, the diameter of the articulating surfaces can be maximised whilst retaining sufficient resistance to brittle fracture. The diameter of articulating surfaces for a ceramic acetabular cup is limited by the comparatively thick wall of the acetabular cup and supporting construction that are required if the cup is formed from a ceramic material. In contrast, a metallic acetabular cup having at least a portion of a surface comprising a ceramic material can permit a larger articulation, while maintaining sufficient resistance to brittle fracture. This is an important advantage of the invention, as the benefits of a large articulation, notably increased stability and range of motion and reduced risk of dislocation, are widely recognised. In particular, the present invention facilitates fabrication of a modular acetabular cup for femoral surface replacement. Such a cup cannot be fabricated from ceramic material, as it would require excessive reaming of either the acetabulum or the femoral head.

According to another aspect of the present invention, there is provided a method of forming a prosthesis having first and second articulating components, comprising: forming a first component from a ceramic material, forming a second component from a metallic material, and modifying the second component such that at least a portion of a surface of the second component comprises a coating of ceramic material of 2 to 12 microns in thickness, the coating comprising a multilayer superlattice coating applied by high power impulse magnetron sputtering.

The coating may comprise one or more materials selected from the group comprising: diamond like carbon, titanium nitride, titanium niobium nitride, titanium carbonitride, chromium nitride, chromium carbonitride, chromium carbide, chromium oxide, titanium aluminium nitride, niobium nitride and niobium carbide.

### BRIEF DESCRIPTION OF THE DRAWINGS

For a better understanding of the present invention, and to show more clearly how it may be carried into effect, reference will now be made, by way of example, to the accompanying drawings, in which:-

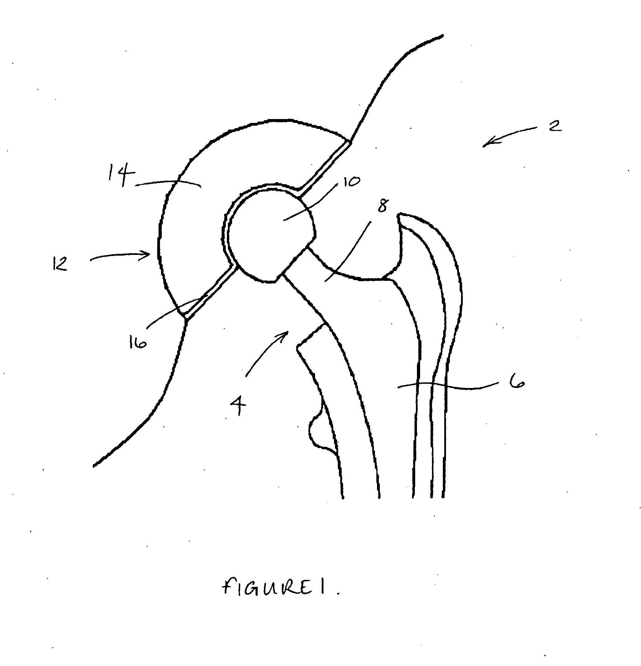

Figure 1 is a sectional view of a hip prosthesis.

### DESCRIPTION OF PREFERRED EMBODIMENTS

A hip replacement prosthesis 2 includes a femoral component 4, comprising a stem 6, a neck 8 and a femoral head 10, and an acetabular component 12 comprising an acetabular cup 14. The femoral component 4 may comprise a single component or may be modular, having for example a tapered metal sleeve. According to one embodiment of the present invention, at least the head 10 of the femoral component is formed from a ceramic material. Preferred materials for the femoral head include alumina, stabilised zirconia, zirconia toughened alumina, silicon nitride and silicon carbide. However, the femoral head may be formed from any other suitable biocompatible ceramic material.

The acetabular cup 14 is formed from a metallic material. Preferred materials include cobalt chromium based alloys, titanium alloys and stainless steels. Most preferred is a cobalt chromium alloy conforming to ASTM F-1537 or ASTM F75. The cup may be coated on a bone contacting surface with hydroxyapatite (HA) or other material to support bone ingrowth. The cup may comprise a single component or may include a shell and separate liner received within the shell. A bearing surface 16 of the acetabular cup 14 comprises a ceramic material.

According to a first preferred embodiment, the ceramic material of the bearing surface is in the form of a coating applied to the bearing surface. The bearing surface contacts the femoral head when the prosthesis is assembled. The coating covers the entire bearing surface and may extend to cover the entire component. Preferred coatings for the acetabular cup include films produced from single or multilayer combinations of one or more of the following classes of materials: diamond like carbon, metal nitrides, metal oxides or metal carbines. Suitable example materials include diamond like carbon, titanium nitride, titanium niobium nitride, titanium carbonitride, chromium nitride, chromium carbonitride, chromium carbide, chromium oxide, titanium aluminium nitride, niobium nitride and niobium carbide. The coating is preferably formed on the acetabular cup by a vapour deposition technique such as cathodic arc evaporation, pulsed laser deposition, electron beam evaporation, ion beam assisted deposition, magnetron sputtering or High Power Impulse Magnetron Sputtering (HIPIMS).

The coating may be a single layer coating or may be a multilayer coating. A single layer coating can be formed using established methods, the technology for which is widely available on an industrial scale. Suitable single layer coating materials include titanium nitride, titanium aluminium nitride, chromium nitride and diamond like carbon.

Nanostructured multilayer coatings of ceramic materials are an emerging class of superhard coatings that have particular application to the present invention. In multilayered coatings, repeating layered structures of two or more different materials with nanometre scale dimensions are deposited onto a surface. The nanostructured multilayer coatings are commonly known as "superlattices" and can be considered as new materials with properties, such as hardness, that greatly exceed those of single layered structures produced with the same individual materials. The properties of superlattice coatings may be tailored to the requirements of a given application, through appropriate choice of constituent materials, layer thickness and deposition parameters. Such coatings are therefore particularly preferable for use with the present invention. Example material combinations for multilayered coatings include titanium nitride / niobium nitride and titanium nitride / chromium nitride.

The physical properties of the coating applied to the acetabular cup are dependent upon the type of coating and the materials and methods employed. The thickness of the coating is preferably 8 to 12 microns and most preferably approximately 10 microns for a single layer coating and preferably 2 to 7 microns and most preferably approximately 5 microns for a multilayer coating. Hardness of the coating ranges from approximately 2000HV to 9000HV. Surface roughness of the coating is dependent upon the coating method employed and the surface roughness of the underlying substrate. For example, cathodic arc deposition produces droplets that can increase surface roughness by up to a factor of ten. Arc deposited parts must therefore be polished to remove surface asperities, a process which may be conducted using diamond lapping paste. Due to the hardness of the ceramic surface, polishing results in a surface that is at least as good as the original surface. In contrast, HIPIMS results in only a slight increase in surface roughness and is considerably better than cathodic arc sputtering. Polishing of coatings produced by HIPIMS may therefore not be necessary.

The preferred method of coating the acetabular cup is High Power Impulse Magnetron Sputtering (HIPIMS), as this enables the creation of carefully controlled, macrodefect free single and multilayer structures. HIPIMS produces a highly ionized plasma but, as the technology is based on sputtering, the plasma is essentially droplet free. The resulting coating is smooth and wear resistant with higher density than a coating produced by conventional magnetron sputtering and with fewer droplets / defects than a coating produced by arc evaporation.

According to another preferred embodiment of the present invention, the ceramic material on the bearing surface of the acetabular cup comprises a surface layer formed by chemical and/or structural modification of the metallic surface of the component. The surface layer is formed by plasma diffusion or by ion implantation.

Plasma diffusion introduces species such as nitrogen, carbon and boron into the surface of a work piece by bringing an activated nitrogen, carbon or boron bearing gas into direct contact with the surface at elevated temperature. Nitriding, carburizing and boriding with positive ions derived from the plasma of an electrical glow discharge produces wear, fatigue and corrosion resistant hard surfaces.

Technological variants of the conventional plasma nitriding process like low pressure nitriding and bias free nitriding, nitrocarburizing and plasma nitriding followed by post oxidation processes are all suitable for use with the present invention.

Plasma diffusion results in a uniform build up of compound material, which is hard but still ductile. The component retains its core hardness and there are no resulting dimensional changes of the work piece. Plasma diffusion also has the advantage that a wide range of treatment temperatures are available, the process is non-toxic and environmentally clean, has low energy consumption and can be conducted in a single step operation.

A work piece is placed in a furnace and a low vacuum is obtained using a pump. A high voltage is applied between the work piece and the furnace using pulsed D.C. current supply leading to formation of a plasma. Appropriate selection of current, voltage and applied gas composition results in build up of a compound surface layer having the desired properties. In a preferred embodiment, the applied gas is nitrogen bearing and the work piece is formed of a cobalt chromium based alloy, resulting in a chromium nitride surface layer.

lon implantation is a type of plasma diffusion carried out over a localised area. A beam of ions is accelerated to high speeds through an electric field. The beam is focused on a work piece, penetrating the surface and causing mechanical and chemical changes in the surface layer. The changes are controlled by precise variation of beam intensity.

Each of the preferred embodiments of the invention results in a prosthesis having a ceramic on ceramic articulation, but in which one of the prosthesis components is substantially metallic. The ceramic on ceramic articulation is formed by modifying the bearing surface of the metallic component, either by coating the surface or by conducting one or more surface hardening procedures whereby a ceramic compound surface layer is formed.

The preferred methods of modifying the bearing surface of the metallic component may be combined, resulting in a hybrid process. For example, plasma nitriding may be combined with an additional chromizing step or with the application of a physical vapour deposition coating in order to improve corrosion resistance. Such a hybrid process is known as a Duplex Treatment.

The present invention may be applied to other types of prosthesis having first and second components that are adapted to articulate against each other. For example, the present invention may be applied to knee and shoulder prostheses.

## Claims

1. A prosthesis comprising a first component and a second component, the first component being adapted to articulate with the second component and being formed from a ceramic material, and the second component being formed from a metallic material, wherein at least a portion of a surface of the second component comprises a coating of ceramic material of 2 to 12 microns in thickness applied to the second component, the coating comprising a multilayer superlattice coating applied by high power impulse magnetron sputtering.

2. A prosthesis as claimed in claim 1, wherein the first and second components comprise bearing surfaces.

3. A prosthesis as claimed in claim 2, wherein at least a portion of the bearing surface of the second component comprises a coating of ceramic material.

4. A prosthesis as claimed in claim 2 or 3, wherein the entire bearing surface of the second component comprises a coating of ceramic material.

5. A prosthesis as claimed in any one of the preceding claims, wherein the entire surface of the second component comprises a coating of ceramic material.

6. A prosthesis as claimed in any one of the preceding claims, wherein the first component is formed from a material selected from the group comprising: alumina, stabilised zirconia, zirconia toughened alumina, silicon nitride and silicon carbide.

7. A prosthesis as claimed in any one of the preceding claims, wherein the second component is formed from a material selected from the group comprising: cobalt chromium based alloys, titanium alloys and stainless steels.

8. A prosthesis as claimed in any one of the preceding claims, wherein the portion of a surface of the second component comprises a coating of a chromium compound.

9. A prosthesis as claimed in claim 1, wherein the coating is formed from one or more materials selected from the group comprising: diamond like carbon, titanium nitride, titanium niobium nitride, titanium carbonitride, chromium nitride, chromium carbonitride, chromium carbide, chromium oxide, titanium aluminium nitride, niobium nitride and niobium carbide.

10. A prosthesis as claimed in any one of the preceding claims comprising a hip replacement prosthesis.

11. A prosthesis as claimed in claim 10, wherein the first component comprises a femoral head.

12. A prosthesis as claimed in claim 11, wherein the second component comprises an acetabular cup.

13. A method of forming a prosthesis having first and second articulating components, comprising:
forming a first component from a ceramic material,
forming a second component from a metallic material, and
modifying the second component such that at least a portion of a surface of the second component comprises a coating of ceramic material of 2 to 12 microns in thickness, the coating comprising a multilayer superlattice coating applied by high power impulse magnetron sputtering.

14. A method as claimed in claim 13, wherein the coating comprises one or more materials selected from the group comprising: diamond like carbon, titanium nitride, titanium niobium nitride, titanium carbonitride, chromium nitride, chromium carbonitride, chromium carbide, chromium oxide, titanium aluminium nitride, niobium nitride and niobium carbide.

## Patentansprüche

1. Prothese, die eine erste Komponente und eine zweite Komponente umfasst, wobei die erste Komponente derart ausgelegt ist, dass sie mit der zweiten Komponente ein Gelenk bildet, und aus einem Keramikmaterial hergestellt ist und die zweite Komponente aus einem Metallmaterial hergestellt ist, wobei mindestens ein Abschnitt einer Oberfläche der zweiten Komponente eine Beschichtung aus Keramikmaterial mit 2 bis 12 µm Dicke umfasst, die auf die zweite Komponente aufgebracht worden ist, wobei die Beschichtung eine durch Hochleistungs-Impuls-Magnetron-Sputtern aufgebrachte mehrschichtige Supergitter-Beschichtung umfasst.

2. Prothese nach Anspruch 1, wobei die erste Komponente und die zweite Komponente Lagerflächen umfassen.

3. Prothese nach Anspruch 2, wobei mindestens ein Abschnitt der Lagerfläche der zweiten Komponente eine Beschichtung aus Keramikmaterial umfasst.

4. Prothese nach Anspruch 2 oder 3, wobei die gesamte Lagerfläche der zweiten Komponente eine Beschichtung aus Keramikmaterial umfasst.

5. Prothese nach einem der vorhergehenden Ansprüche, wobei die gesamte Oberfläche der zweiten Komponente eine Beschichtung aus Keramikmaterial umfasst.

6. Prothese nach einem der vorhergehenden Ansprüche, wobei die erste Komponente aus einem Material hergestellt wird, das ausgewählt ist aus der Gruppe mit: Aluminiumoxid, stabilisiertem Zirkonoxid, Zirkonoxid-verstärktem Aluminiumoxid, Siliziumnitrid und Siliziumcarbid.

7. Prothese nach einem der vorhergehenden Ansprüche, wobei die zweite Komponente aus einem Material hergestellt wird, das ausgewählt ist aus der Gruppe mit: Legierungen auf Kobalt/Chrom-Basis, Titanlegierungen und nichtrostenden Stählen.

8. Prothese nach einem der vorhergehenden Ansprüche, wobei der Abschnitt einer Oberfläche der zweiten Komponente eine Beschichtung aus einer Chromverbindung umfasst.

9. Prothese nach Anspruch 1, wobei die Beschichtung aus ein oder mehr Materialien hergestellt wird, ausgewählt aus der Gruppe mit: diamantartigem Kohlenstoff, Titannitrid, Titanniobnitrid, Titancarbonitrid, Chromnitrid, Chromcarbonitrid, Chromcarbid, Chromoxid, Titanaluminiumnitrid, Niobnitrid und Niobcarbid.

10. Prothese nach einem der vorhergehenden Ansprüche, die eine Hüftersatzprothese umfasst.

11. Prothese nach Anspruch 10, wobei die erste Komponente einen Femurkopf umfasst.

12. Prothese nach Anspruch 11, wobei die zweite Komponente eine Hüftpfanne (Acetabulum) umfasst.

13. Verfahren zur Herstellung einer Prothese mit einer ersten und einer zweiten Komponente, die miteinander ein Gelenk bilden, umfassend:
Herstellen einer ersten Komponente aus einem Keramikmaterial,
Herstellen einer zweiten Komponente aus einem Metallmaterial und
Modifizieren der zweiten Komponente derart, dass mindestens ein Abschnitt einer Oberfläche der zweiten Komponente eine Beschichtung aus Keramikmaterial mit 2 bis 12 µm Dicke umfasst, wobei die Beschichtung eine durch Hochleistungs-Impuls-Magnetron-Sputtern aufgebrachte mehrschichtige Supergitter-Beschichtung umfasst.

14. Verfahren nach Anspruch 13, wobei die Beschichtung ein oder mehr Materialien umfasst, ausgewählt aus der Gruppe mit: diamantartigem Kohlenstoff, Titannitrid, Titanniobnitrid, Titancarbonitrid, Chromnitrid, Chromcarbonitrid, Chromcarbid, Chromoxid, Titanaluminiumnitrid, Niobnitrid und Niobcarbid.

## Revendications

1. Prothèse comprenant un premier composant et un deuxième composant, lequel premier composant, adapté pour s'articuler avec le deuxième composant, est constitué d'un matériau céramique et lequel deuxième composant est constitué d'un matériau métallique, dans laquelle prothèse au moins une partie d'une surface du deuxième composant est dotée d'un revêtement de matériau céramique appliqué sur le deuxième composant, épais de 2 à 12 micromètres, lequel revêtement comprend un revêtement multicouche super-réseau, appliqué par pulvérisation magnétron à impulsions de haute énergie.

2. Prothèse conforme à la revendication 1, dans laquelle le premier composant et le deuxième composant comportent des surfaces d'appui.

3. Prothèse conforme à la revendication 2, dans laquelle au moins une partie de la surface d'appui du deuxième composant est dotée d'un revêtement de matériau céramique.

4. Prothèse conforme à la revendication 2 ou 3, dans laquelle la totalité de la surface d'appui du deuxième composant est dotée d'un revêtement de matériau céramique.

5. Prothèse conforme à l'une des revendications précédentes, dans laquelle la totalité de la surface du deuxième composant est dotée d'un revêtement de matériau céramique.

6. Prothèse conforme à l'une des revendications précédentes, dans laquelle le premier composant est constitué d'un matériau choisi parmi les suivants : alumine, zircone stabilisée, alumine renforcée par zircone, nitrure de silicium et carbure de silicium.

7. Prothèse conforme à l'une des revendications précédentes, dans laquelle le deuxième composant est constitué d'un matériau choisi parmi les suivants : alliages à base de cobalt et de chrome, alliages de titane, et aciers inoxydables.

8. Prothèse conforme à l'une des revendications précédentes, dans laquelle ladite partie d'une surface du deuxième composant est dotée d'un revêtement formé d'un composé du chrome.

9. Prothèse conforme à la revendication 1, dans laquelle ledit revêtement est formé d'un ou plusieurs matériau(x) choisi(s) parmi les suivants : carbone diamantaire, nitrure de titane, nitrure de niobium et de titane, carbonitrure de titane, nitrure de chrome, carbonitrure de chrome, carbure de chrome, oxyde de chrome, nitrure d'aluminium et de titane, nitrure de niobium, et carbure de niobium.

10. Prothèse conforme à l'une des revendications précédentes, comprenant une prothèse de hanche.

11. Prothèse conforme à la revendication 10, dans laquelle le premier composant comprend une tête de fémur.

12. Prothèse conforme à la revendication 11, dans laquelle le deuxième composant comprend un cotyle.

13. Procédé de production d'une prothèse comportant un premier composant et un deuxième composant s'articulant l'un avec l'autre, lequel procédé comporte les étapes suivantes :
- former un premier composant en un matériau céramique ;
- former un deuxième composant en un matériau métallique ;
- et modifier le deuxième composant de telle sorte qu'au moins une partie d'une surface du deuxième composant soit dotée d'un revêtement de matériau céramique épais de 2 à 12 micromètres, ce revêtement comprenant un revêtement multicouche super-réseau, appliqué par pulvérisation magnétron à impulsions de haute énergie.

14. Procédé conforme à la revendication 13, dans laquelle ledit revêtement comprend un ou plusieurs matériau(x) choisi(s) parmi les suivants : carbone diamantaire, nitrure de titane, nitrure de niobium et de titane, carbonitrure de titane, nitrure de chrome, carbonitrure de chrome, carbure de chrome, oxyde de chrome, nitrure d'aluminium et de titane, nitrure de niobium, et carbure de niobium.
